(19) Europäisches Patentamt
European Patent Office
Office européen des brevets

(11) EP 1 268 592 B1

(12) EUROPEAN PATENT SPECIFICATION

(45) Date of publication and mention of the grant of the patent:
26.10.2005 Bulletin 2005/43

(51) Int Cl.7: $C08G\ 12/42$, C07D 251/64, C08K 5/3492

(21) Application number: 01953018.7

(22) Date of filing: 14.02.2001

(86) International application number:
PCT/US2001/004596

(87) International publication number:
WO 2001/060882 (23.08.2001 Gazette 2001/34)

(54) **ALKOXYMETHYL MELAMINE CROSSLINKERS**

ALKOXYMETHYLMELAMIN VERNETZER

AGENTS DE RETICULATION D'ALCOXYMETHYL MELAMINE

(84) Designated Contracting States:
AT BE CH CY DE DK ES FI FR GB GR IE IT LI LU MC NL PT SE TR

(30) Priority: 15.02.2000 US 182630 P

(43) Date of publication of application:
02.01.2003 Bulletin 2003/01

(73) Proprietor: CYTEC SURFACE SPECIALTIES, S.A.
1070 Bruxelles (BE)

(72) Inventors:
• SPITZ, George, T.
Longmeadow, MA 0116 (US)
• VAUGHN, George, D.
Southwick, MA 01077-9666 (US)
• BALL, Patricia, A.
Chicopee, MA 01013 (US)

(74) Representative: Deckers, Hellmuth Alexander
European Patent Attorney,
Bahnhofstrasse 26A
55218 Ingelheim (DE)

(56) References cited:
EP-A- 0 374 340     EP-A- 0 668 334
US-A- 4 433 143     US-A- 4 575 536
US-A- 5 939 195     US-A- 5 981 074

## Description

### Related Application

This application claims the benefit of priority under 35 U.S.C. § 119(e) of U.S. Provisional Application Serial No. 60/182,630, filed February 15, 2000.

### Field of Invention

Disclosed herein are alkoxymethyl melamine crosslinkers and methods of making and using such melamine crosslinkers in coating compositions.

### Background

Melamine-formaldehyde amino resins are used in many, thermally-cured, industrial coating applications, e. g. wood, automotive, appliances, coil, etc., as crosslinkers to form coatings comprising a three-dimensional polymer network. Functional groups of melamine-formaldehyde amino resins can react with functional groups on the vehicle resins as well as with themselves in a self-condensation. Melamine-formaldehyde amino resins are mixtures of monomeric and oligomeric molecules. The melamine unit comprises a heterocyclic six-membered ring of alternating carbon and nitrogen atoms where each of the three ring carbon atoms has a pendant (non-ring) nitrogen atom which can link to two functional groups. Thus, each monomeric melamine unit has six functional groups.

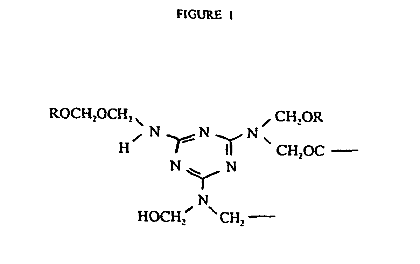

In the production of melamine formaldehyde amino crosslinker resins, melamine is reacted with formaldehyde to theoretically form hexamethylol melamine which is reacted with an alcohol, e.g. methanol or mixtures of methanol and, say, butanol, to theoretically form a hexa alkoxymethyl melamine. An object of such reaction is hexa alkoxymethyl melamine. In fact, the reaction provides a variety of functional groups attached to the pendant nitrogen atoms including some of which represent bridges between triazine units. With reference to Figure 1 there is shown a hypothetical melamine molecule with a different functional group on each of the non-ring nitrogen atoms. Clockwise are imino (>N-H), acetal (>N-CH$_2$OCH$_2$OR), alkoxymethyl (>N-CH$_2$OR), methylene ether bridge (>N-CH$_2$-O-CH$_2$-N<), methylene bridge (>N-CH$_2$-N<) and methylol (>N-CH$_2$OH) groups. The methylene ether bridge and methylene bridge link melamine units into oligomeric species, e.g. dimers, trimers, etc.

Santer et al. disclose in U. S. Patent 4,433,143 a methylated methylolated melamine composition which has a combined formaldehyde content stated as ratio of formaldehyde to melamine in the range of 2.4 mol/mol to 3.2 mol/mol, and a combined methanol content stated as ratio of methanol to melamine of from 2.3 mol/mol to 3.0 mol/mol, and stated as ratio of methanol to formaldehyde of from 0.7 mol/mol to 1.0 mol/mol. No ranges for imino content nor a relation of this content to the oligomer content are stated.

Erikson et al. disclose in U.S. Patent 3,322,762 the production of "substantially hexakis(methoxymethyl)melamine" having nitrogen values within 0.2 percent of theoretical. Erikson et al. teach reacting substantially hexamethylol melamine with excess methanol, at least 20 moles of methanol per mole of melamine, at pH between about 1 and 3 to produce a "fully methylated" hexakis(methoxymethyl)melamine. The product can be dissolved in water in amount of up to 33 percent product in water at 25 °C.

Samaraweera et al. in *J. Appl. Polymer Sci*. Vol. 45, 1903-1909 (1992) reported the extraction of 90-95% pure hexa methoxymethyl melamine from an commercial melamine resin resulting in a product which was estimated by size exclusion chromatography to be 95% monomer and 5% dimer. It is reported that reactivity of commercial melamine resins is enhanced by removal of all active hydrogen species. The use of the extracted melamine in coatings was reported by Chu et al. in *J. Coatings Tech.,* Vol. 65, No. 819 (April 1993). However, the extraction procedures of Samaraweera et al. are inefficient resulting in only 14% yield from a commercial melamine formaldehyde resin.

Graziano et al. disclose in US Patent 5,376,504 the recovery of monomeric hexamethoxymethyl melamine from byproducts of synthesis by batch distillation, e.g. of Cymel 300 (commercially available from Cytec, Inc.) for use in preparing photoresists having improved sensitivity and shelf life. There is no indication of recovery efficiency. Because oligomeric derivatives of alkoxymethyl melamine are useful crosslinking agents, there is little incentive to develop processes for the manufacture of monomeric alkoxymethyl melamine products.

Cameron et al. in US Patent 4,837,278 reports the use of a "low imino, fully alkylated aminoplast crosslinking agent. The preferred aminoplast resin is reportedly supplied by Monsanto Corporation under the designation Resimene RF-4518. Recent analysis of retained samples of Resimene 4518 show the aminoplast to be a mixed alkoxymethyl melamine comprising methoxymethyl and 2-ethylhexoxymethyl groups, typically comprising about 5.0 alkoxymethyl groups per mole of melamine with an imino level of about 0.28 wt. %. and a low monomer content of about 30%.

An object of this invention is to provide improved alkoxymethyl melamine compositions for use as low temperature crosslinkers in the production of flexible, tough coatings. Another object is to provide improved alkoxymethyl melamine compositions with reduced catalyst demand. Another object is to provide improved low temperature curing alkoxymethyl melamine compositions which provide coatings with improved solvent resistance. These and other objects of the invention as will be apparent from the following disclosed of detailed description and examples are achieved by providing a mixture of monomeric and oligomeric alkoxymethyl melamine compounds with high alkyl substitution and low imino content.

## Summary of the Invention

This invention provides aminoplast crosslinker compositions of alkoxymethyl melamine derivatives comprising up to 95 percent by weight of monomeric alkoxymethyl melamine with at least 5.0 moles of alkoxymethyl groups attached to pendant nitrogen atoms of said melamine and a low level of imino groups, e.g. less than 0.7 percent by weight (wt. %) of the alkoxymethyl melamine derivatives of imino groups (>N-H).

In one preferred aspect of the invention the alkoxymethyl melamine derivatives have a lower level of imino groups e.g. less than 0.2 wt.% of imino groups.

In another aspect of the invention the crosslinker compositions comprise methoxymethyl melamine derivative with at least 5.0 moles of methoxymethyl groups attached to pendant nitrogen atoms of said melamine.

In another aspect of the invention the crosslinker compositions comprise alkoxymethyl melamine derivatives with at least 5.6 moles of alkoxymethyl groups attached to pendant nitrogen atoms of said melamine where the alkoxymethyl groups are mixtures of methoxymethyl and minor amounts higher alkoxymethyl groups.

In another aspect of the invention the crosslinker compositions comprise methoxymethyl derivatives comprising a high level of oligomers, e.g. between about 30 and 50 weight percent of dimers, trimers and higher oligomeric forms of methyoxymethyl melamine. Such methoxymethyl melamine derivatives are preferably in a liquid form at room temperatures, e.g. 20-25 °C.

## Brief Description of the Drawings

Figure 1 is a structural representation of a melamine derivative with a variety of functional groups attached to pendant nitrogen atoms.

Figure 2 is a compositional map of melamine derivatives expressed in terms of imino and oligomer content.

Figure 3 is a set of chromatographic analyses illustrating the separation of monomer and oligomeric content of an alkoxymethyl melamine composition.

## Detailed Description of Preferred Embodiments

As used herein the term "oligomer content" refers to the weight percent of dimers, trimers and higher oligomeric forms of alkoxymethyl melamine derivatives, e.g. as determined by size exclusion chromatography.

As used herein the term "imino content" refers to the weight percent of imino units per alkoxymethyl melamine compound, e.g. as determined by hydrogen nuclear magnetic resonance ($^1$H-NMR) analysis. A preferred and reliable method for $^1$H-NMR analysis uses a 5 mm solution probe on a Bruker AC-200 NMR analyzer with measurements performed spinning at 20 Hz at 30°C, using a 4 second acquisition time, 32 scans, and a 22.5 second recycle delay at a spectral frequency of 200.13 MHz. All samples are analyzed in duplicate. A hexamethyldisiloxane (HMDS) standard stock solution (0.05 wt. %. HMDS) is prepared by dissolving 100 mg of HMDS in acetone-$d_6$. Samples are prepared by dissolving approximately 20 mg of melamine compound in nominally 500 μl of the stock solution (measured values were weight/weight, not weight/volume). Spectral referencing is done by setting the methyl peak of HMDS referenced to 0.0 ppm. "Imino content" is quantitated via the >NH peak at 7.0 ppm (integrated from 7.3 to 6.7 ppm) while the amount of HMDS internal standard is quantitated via the methyl peak at 0.0 ppm (integrated from 0.3 to -0.3 ppm). The following equations are used to calculate the amount of internal standard HMDS in the sample, the moles of HMDS in the sample and the moles of imino in the sample:

$$\text{Moles of HMDS} = (\text{Mass of stock solution}) \, (0.0005)/162.38 \text{ g/mol} \qquad \text{(Equation 1)}$$

$$\text{Moles of NH} = (\text{Area of NH}) \, (18) \, (\text{Moles of HMDS}) / (\text{Area of HMDS}) \qquad \text{(Equation 2)}$$

$$\text{imino content} = \text{(Moles of NH) (15.01 g/mol) / (Mass of sample)} \qquad \text{(Equation 3)}$$

The factor of 162.38 is the molecular weight of HMDS; 18 represents the number of methyl protons per mole of HMDS; and, 15.01 is the molecular weight of an imino group. The alkoxymethyl melamine derivatives of this invention comprise a mixture of functional groups attached to the pendant nitrogen atoms. Preferably the predominant functional group is an alkoxymethyl group ($>N-CH_2OR$) with lesser amounts of imino ($>N-H$), methylol ($>N-CH_2OH$), acetal ($>N-CH_2OCH_2OR$), methylene ($>N-CH_2-N<$) and alkoxymethyl methylene ether ($>N-CH_2-O-CH_2-N<$) groups. The methylene and alkoxymethyl methylene ether groups serve to bridge melamine units into oligomers, e.g. dimers, trimers and higher oligomer units. The predominant alkoxymethyl groups are selected from the group consisting of methoxymethyl groups and mixtures of methoxymethyl and higher alkoxymethyl groups.

The alkoxymethyl melamine derivatives in the compositions of this invention comprise a low level of imino groups, e.g. less than 0.7 wt. % of imino groups per unit mass of alkoxymethyl melamine derivatives. In preferred aspects of the invention the alkoxymethyl melamine derivatives have an even lower level of imino groups e.g. less than 0.6 wt. % or less, say, less than 0.5 wt. % of imino groups. In even more preferred aspects of the invention the alkoxymethyl melamine derivatives will comprise less than 0.4 wt. % or lower, say, less than 0.3 wt. % of imino groups. In even more preferred aspects of this invention the alkoxymethyl melamine derivatives will comprise less than 0.2 wt. % of imino groups.

Another aspect of this invention provides alkoxymethyl melamine derivatives with a 20-30 wt. % oligomer level having an imino content, I, defined by the algorithm, $I \geq 0.02X - 0.2$, where X is the weight percent oligomer in the composition and I is expressed in weight percent imino.

Another aspect of this invention provides methoxymethyl melamine derivatives with a high level of oligomers, e.g. greater than about 30 wt. % oligomer, preferably not more than 60 wt. % oligomer, e.g. about 30 to 50 wt. % oligomer. In a preferred aspect of the high oligomeric melamine derivatives, the compositions are liquid at typical room temperatures, e.g. in the range of 20-25 °C. Such liquid methoxymethyl melamine derivatives will typically have an imino content of at least 0.2 wt. % and less than 0.7 wt. %, preferably between 0.2 and 0.5 wt. %, more preferably between 0.2 and 0.4 wt. %, and even more preferably between about 0.2 and 0.3 wt. %.

Another aspect of this invention provides methoxymethyl melamine derivatives with a high level of oligomers, e.g. greater than about 30 wt. % oligomer, preferably not more than 60 wt. % oligomer, e.g. about 30 to 50 wt. % oligomer. In another preferred aspect of the high oligomeric melamine derivatives, the compositions are solid at typical room temperatures, e.g. in the range of 20-25 °C. Such solid methoxymethyl melamine derivatives will typically have an imino content of less than about 0.2 wt. %.

Still another aspect of this invention provides alkoxymethyl melamine derivatives of low oligomeric content, e.g. less than 30 wt. % oligomer and low imino content. e.g. less than about 0.2 wt. %. When such alkoxymethyl melamines comprise mixed alkoxy groups, e.g. primarily methoxymethyl groups with a low level of higher alkoxymethyl groups, the melamine derivatives tend to be advantageously liquid at room temperatures. In another preferred aspect of this invention the alkoxymethyl melamine derivatives comprise a mixture of methoxymethyl and higher alkoxymethyl groups attached to pendant nitrogen atoms. The incorporation of only a minor amount, e.g. about 0.2 to 2 moles of higher alkoxymethyl groups provides an alkoxymethyl melamine composition which tends to be liquid at room temperature to facilitate preparation of liquid coating compositions. Higher alkoxymethyl groups can comprise, ethoxymethyl, n-propoxymethyl, isopropoxymethyl, n-butoxymethyl, isobutoxymethyl, 2-ethylhexyloxymethyl and the like.

Alkoxymethyl melamines comprising exclusively methoxymethyl melamine derivatives tend to be crystalline solids at room temperatures depending on purity. These solids can advantageously be solubilized, e.g. with lower alcohols such a methanol, ethanol and propanol, to provide a liquid composition to facilitate formulations. In other cases solid methoxymethyl melamine derivative compositions can be dispersed into dry powder coating compositions or into liquid coating compositions using techniques well known to those skilled in the art.

The alkoxymethyl melamine derivatives will preferably comprise at least 5.0 moles of alkoxymethyl groups, primarily methoxymethyl groups, attached to pendant nitrogen atoms for each mole of melamine. More preferably the alkoxymethyl melamine derivatives will comprise at least 5.2 moles of alkoxymethyl groups or higher, e.g. at least about 5.4 moles, or even more preferably at least 5.6 moles of alkoxymethyl groups. In especially preferred embodiments, the alkoxymethyl derivatives will comprise at least 5.7 moles or more, e.g. at least 5.8 moles, of alkoxymethyl groups attached to pendant nitrogen atoms for each mole of melamine.

The compositions of this invention will comprise up to about 95 wt. % of monomeric alkoxymethyl melamine derivatives, e.g. at least about 50 wt. % monomer. The balance of alkoxymethyl melamine derivatives is oligomeric, e.g. dimer, trimer, etc. where the melamine units are bridged, e.g. by methylene units or methylene ether groups. In preferred aspects of this invention the composition will comprise from about 50 to about 90 wt. % monomer, even more preferably more than about 70 wt. % monomer or higher, e.g. at least about 80 wt. % monomer.

The alkoxymethyl melamine derivative components of the compositions of this invention are prepared by first reacting melamine with formaldehyde under alkaline conditions (e.g. at pH ≥ 8) to form methylol-functionalized melamine an intermediate product which is recovered in a dry form without excess heating. For instance, the reaction of formaldehyde and melamine is preferably effected by preparing a heel of hot (at least 37 °C), alkaline (pH >8), concentrated (37 % or higher, e.g. 50-56 %) aqueous formaldehyde in a molar excess, e.g. in the range of 9:1 to about 16:1, of the melamine to be reacted. Additional formaldehyde can be also be added in later process steps to promote alkylation. After addition of melamine the reaction mixture is raised to about 90 °C and maintained at temperature to drive reaction towards completion. The reaction mixture is readily cooled by addition of water, e.g. typically less than 30 °C, to produce a slurry temperature of less than about 40 °C to effect precipitation of methylol melamine. Preferred methods of recovery include filtration or centrifugation followed by drying the solids, e.g. in a tray, fluid bed or rotary dryer. Prior art practices such as distillation are likely to result in redissolution and/or resinification of the methylol melamine.

Alternatively, the process can be simplified if water is minimized in the reaction and cooling steps. In place of aqueous formaldehyde, it is preferable to use a mixture of 55 % formaldehyde, 35 % methanol and 10 % water, e.g. commonly available under the tradename of methyl Formcel (available from Celanese Corporation). Even less water is possible in the reaction mixtures by using a mixture of paraformaldehyde dissolved in methanol. The methylol melamine can be prepared as a wet slurry in methanol. See, for instance, US Patent 3,322,762.

The methylol-functionalized melamine is reacted with methanol under acidic conditions (e.g. at pH ≤ 5) to form methyoxymethyl-functionalized melamine in an etherification reaction. Excess formaldehyde and alcohol are critical for obtaining the high degree of alkylation desired in the melamine derivatives of this invention. The high level of alkylation is achieved through multiple reaction steps with spent reactants, e.g. alcohols and water, removed between reaction steps. A dry methylol-functionalized melamine product can be reacted with methanol adjusted to an acidic pH (e.g. pH <5) with nitric acid at a temperature in the range of about 25 to 70 °C to produce a methoxymethyl-functionalized melamine. Because etherification proceeds partially at best, the degree of alkylation can be improved by carrying out the etherification reactions in multiple steps with the addition of formaldehyde and removal of water, along with spent alcohol and uncombined formaldehyde between etherification reactions. Removal is effected by distillation, e.g. vacuum distillation, and the pH is preferably adjusted to a basic condition, e.g. pH ≥ 8, to stop the reaction and minimize formation of oligomers. In the subsequent etherification steps preferably the molar ratio of excess methanol to melamine is at least about 10:1, pH is less than 5, and reaction temperature is in the range of about 25 to 70 °C. Removal of byproducts and unreacted materials especially formaldehyde is effected after all reactions are completed by distillation at pH of at least 8 to a temperature of 120 °C at full vacuum. The product is filtered to remove byproduct salts and is recovered as a clear, colorless liquid which may crystallize to a waxy solid at room temperature depending on oligomer content. This product is useful as a methoxymethyl-functionalized melamine derivative crosslinker in coating compositions. Preferably the products of the above reactions provide a low imino and methylol content, e.g. less than 0.4 wt. % of imino groups per unit mass of melamine derivatives, a high degree of methylation, e.g. greater than 5.0 moles of methoxymethyl per mole of melamine and a high degree of monomer content, e.g. greater than 60 wt. % monomer.

Furthermore, commercially available alkoxymethyl melamine resins can be alkylated to a higher degree to similar advantage by using these alkoxymethyl melamine derivatives as a starting material. Formaldehyde solution is added to the melamine resin, and then distilled under vacuum at pH > 8 to further methylolate the resin. The resulting adduct is etherified with alcohol, preferably methanol, as described above. These reactions can also be effected in multiple steps to achieve the high degree of alkylation desired.

Alternatively, all or part of the methanol in the last etherification step can be replaced by alcohols other than methanol. The reaction product of this transetherification is a liquid, mixed ether, alkoxymethyl-functionalized melamine which can be advantageously used in coating compositions.

The crosslinking compositions of this invention can be used with reactive resin vehicle to provide highly crosslinked, durable coating films. Vehicle resins can comprise a variety of reactive groups for condensation reactions with the alkoxymethyl-functionalized melamine crosslinking agent. Such vehicle reactive groups can comprise alcoholic hydroxy, carboxy, amide, mercaptan and urethane groups or mixtures of such reactive groups. Condensation reactions typically require acid catalyst and/or elevated temperature depending on the nature of the reactive groups of the vehicle. Resins containing carboxy or amide groups typically require higher reaction temperature or higher acid catalyst concentration. Preferably the vehicle is a polyol resin containing primary hydroxy groups.

The following examples serve to illustrate how to make certain embodiments of this invention.

Example 1

This example illustrates the preparation of a highly substituted methylated melamine resin.

a. Preparation of methylol melamine:

973 grams of 37 % strength aqueous formaldehyde was charged to a glass reactor equipped with stirrer, thermometer, heating mantle, condenser, charge funnels, distillate receiver, vacuum pump and gauge . The temperature was adjusted to 37 °C, and 1.0 ml of 50 % sodium hydroxide was added, followed by the addition of 126 g of melamine. The mixture was heated to 90 °C over approximately 10 minutes, and held at this temperature for 15 minutes to react the melamine and formaldehyde to form methylol melamine. Next, 2000 g of water at room temperature was added over approximately 40 minutes, cooling the contents to about 40 °C; the mixture became a thick slurry as methylol melamine precipitates. The slurry was allowed to settle at ambient temperature, and the supernatant was decanted. The thickened slurry was next filtered using a Buchner funnel and vacuum filter flask to recover a wet cake, which was rinsed with methanol to displace water. The resulting cake was spread onto a pan to dry at ambient temperature to constant weight. The resulting methylol melamine powder was dry and free flowing.

b. Preparation of methylated melamine resin:

350 g of methanol were charged to the reactor described above, and 180 g of the methylol melamine powder prepared in 1 a, above was added. The slurry was heated to 29 °C, and 8.0 ml of 70 % nitric acid was added. The temperature was maintained between 32 and 38 °C for 25 minutes as the methylol melamines dissolved and was partially etherified with methanol. The batch was neutralized with 50 % sodium hydroxide to a pH of 9.8 to 10.5 to stop the reaction. Next, 192 g of 37 % aqueous formaldehyde was added, and sodium hydroxide was added to adjust the pH to 9.8 - 10.5. The batch was next distilled under vacuum to an endpoint temperature of 80 °C at 24 inches of mercury vacuum, and approximately 517 g of distillate was removed. Next, an additional 350 g of methanol was added, and the temperature was adjusted to 31 °C followed by the addition of 4.4 ml of 70 % nitric acid. The second methylation reaction was maintained at 30 - 34 °C for 15 minutes, after which the batch was neutralized by adding 50 % sodium hydroxide. Once again, 192 g of 37% formaldehyde was added, and the pH is adjusted to 9.8 - 10.5. The batch was distilled to an endpoint of 80 °C at 24 inches mercury vacuum and approximately 510 g of distillate was removed. Next 350 g of methanol was added and the temperature was adjusted to 30 °C, followed by the addition of 4.4 ml of 70 % nitric acid. The third methylation reaction was maintained at 30 - 34 °C for 15 minutes, after which the batch was neutralized with 50 % sodium hydroxide to a pH of 9.8 - 10.5. The batch was distilled under vacuum to an endpoint of 120 °C at 28 inches of mercury vacuum, and then maintained at these conditions for an additional 20 minutes to obtain an essentially 100 % solids product, and yielding 245.6 g of crude resin. The crude resin was then separated from by-product salts in a horizontal leaf pressure filter. The product thus obtained is a clear essentially colorless liquid having a Gardner letter viscosity of O at 25 °C, and crystallized to a waxy solid on standing at ambient temperature. Oligomer content was 14.9 % as measured by size exclusion chromatography. In addition, the composition of this resin was determined by C 13 NMR shows that 6.1 moles of formaldehyde, and 5.8 moles of methanol each was combined per mole of melamine. Furthermore, imino content as measured by proton NMR was 0.13 % by weight.

Example 2

This example illustrates the preparation of a highly substituted methylated/butylated melamine resin. The procedure of Example 1 was followed up except that in the third methylation reaction the methanol was replaced with a mixture of 245 g of methanol, and 109 g of n-butanol. The temperature was adjusted to 32 °C, and 4.4 ml of 70 % nitric acid was added, and the batch was maintained at this temperature for 15 minutes, after which the batch was neutralized with 50 % sodium hydroxide to a pH of 9.8 - 10.5. The batch was distilled under vacuum to an endpoint of 120 °C at 28 inches mercury vacuum, and then held under these conditions for 20 minutes to obtain an essentially 100 % solids product, and yielding 247.4 g of crude resin. The resin was then filtered to remove by-product salts in a horizontal leaf pressure filter. The product thus obtained was a clear liquid having a Gardner letter viscosity between J and K at 25 °C. Oligomer content as measured by size exclusion chromatography was 6.5 wt. %. In addition, the composition of this resin as determined by C13 NMR showed that 6.06 moles of formaldehyde, and 5.37 moles of methanol, and 0.51 moles of butanol each were combined per mole of melamine. Furthermore, imino content as measured by proton NMR was 0.08 wt. %. This resin did not crystallize to a waxy solid, but remained a stable liquid at ambient temperature.

Example 3

A methoxymethyl melamine similar to the product of Example 1 was produced having imino content of 0.11 wt. % and a monomer content of 19 wt. %.

Example 4a

This example illustrates the preparation of a highly substituted methylated melamine resin. Resimene 747 commercially available methylated melamine from Solutia Inc. typically has an imino content of about 1 wt. %, and a composition of about 5.0 moles of methanol combined per mole of melamine. 360.4 g of Resimene 747 methylated melamine was charged to the reactor described in Example 1 along with 149.6 g of 56 % aqueous formaldehyde solution, and the pH was adjusted to 9.5 with sodium hydroxide. The mixture was then distilled under vacuum to an endpoint of 80 °C at 26 inches mercury vacuum. 320 g of methanol was then charged cooling the batch to 56 °C, followed by 5 ml of 35 % nitric acid. After reacting for 20 minutes at 56 - 60 °C, the batch was neutralized with 50 % sodium hydroxide, and it's pH was adjusted to 9.8. The batch was then distilled under vacuum to an endpoint of 120 °C at 28 inches mercury, and maintained at these conditions for 20 minutes to obtain an essentially 100 % solids product, and yielding 369.5 g of crude resin. The crude resin was then filtered to remove by-product salts in a horizontal leaf pressure filter. The product thus obtained was a clear essentially colorless liquid having a Gardner viscosity of Z at 25 °C. Oligomer content was 40 wt. % as measured by size exclusion chromatography. In addition, the composition of this resin as determined by C 13 NMR showed that 5.88 moles of formaldehyde, and 5.28 moles of methanol each were combined per mole of melamine. Furthermore, imino content as measured by proton NMR was 0.47 wt. %.

Examples 4b-d

These examples further illustrate the preparation of a more highly substituted methylated melamine resin generally according to the method of Example 4a. Preparation 4b provided a methoxymethyl melamine having an imino content of 0.21 wt. % and an oligomer content of 42 wt. %. Preparation 4c provided a methoxymethyl melamine having an imino content of 0.23 wt. % and an oligomer content of 47 wt. %. Preparation 4d provided a methoxymethyl melamine having an imino content of 0.66 wt. % and an oligomer content of 40 wt. %.

Example 5a

This example illustrates the preparation of a more highly substituted methylated melamine resin than in Examples 4 a-d. This is accomplished by adding an additional methylation step. 361.2 g of Resimene 747 methylated melamine resin having an imino content greater than about 1 wt. %, was charged to the reactor described in Example 1 along with 160 g of 56 % strength aqueous formaldehyde solution, and the pH was adjusted to 10.9 with sodium hydroxide. The mixture was next distilled under vacuum to an endpoint of 75 °C at 24 inches mercury vacuum. 320 g of methanol was then charged cooling the batch to 56 °C, followed by 3.5 ml of 35 % nitric acid. After reacting for 18 minutes at 56 - 58 °C, the batch was neutralized with 50 % sodium hydroxide, and its pH was adjusted to 9.5. The batch was next distilled under vacuum to an endpoint of 80 °C at 26 inches mercury vacuum.
The intermediate methoxymethyl melamine product was mixed with 320 g of methanol and the mixture cooled to 57 °C. Next, 3.5 ml of 35 % nitric acid was charged. After reacting for 20 minutes at 57 - 58 °C, the batch was neutralized with 50 % sodium hydroxide, and it's pH was adjusted to 9.3. The batch was then distilled under vacuum to an endpoint of 120 °C at 28 inches mercury, and maintained at these conditions for 20 minutes to obtain an essentially 100 % solids product, and yielding 373 g of crude resin. The crude resin was then filtered to remove by-product salts in a horizontal leaf pressure filter. The final product thus obtained was a clear essentially colorless liquid having a Gardner viscosity of X-Y at 25 °C. Oligomer content was 36 wt. % as measured by size exclusion chromatography. In addition, the composition of this resin as determined by C13 NMR showed that 5.97 moles of formaldehyde, and 5.62 moles of methanol each were combined per mole of melamine. Furthermore, imino content as measured by proton NMR was 0.36 wt. % by weight. This product crystallized to a waxy solid on standing at ambient temperature, but more slowly than the product of example 1.

Examples 5b-c

These examples further illustrate the preparation of a more highly substituted methylated melamine resin generally according to the method of Example 5a. Preparation 5b provided a methoxymethyl melamine having an imino content of 0.18 wt. % and an oligomer content of 45 wt. %. Preparation 5c provided a methoxymethyl melamine having an imino content of 0.11 wt. % and an oligomer content of 50 wt. %.

Example 6

This example serves to illustrate the preparation of pure monomeric hexamethoxymethyl melamine by separating the monomer from oligomeric species of a methoxymethyl melamine product using a preparative size exclusion chromatography (SEC) column. A mixture of 0.5 g sample of a methoxymethyl melamine and 20 ml of tetrahydrofuran (THF) was shaken in a wrist action shaker for 1 hour. The monomer was separated by size exclusion chromatography using a system comprising a Waters WISP 712 autosampler (2000 µl injection), a Waters 590 programable pump (3 ml/min THF), a Hewlett Packard 1037A refractive index detector (sens = 64) and a Polymer Laboratories Plgel 10 micron, 50A preparative SEC column (300 x 25 mm, part #1210-6115 S/N 10PM-5/1-60A-12).

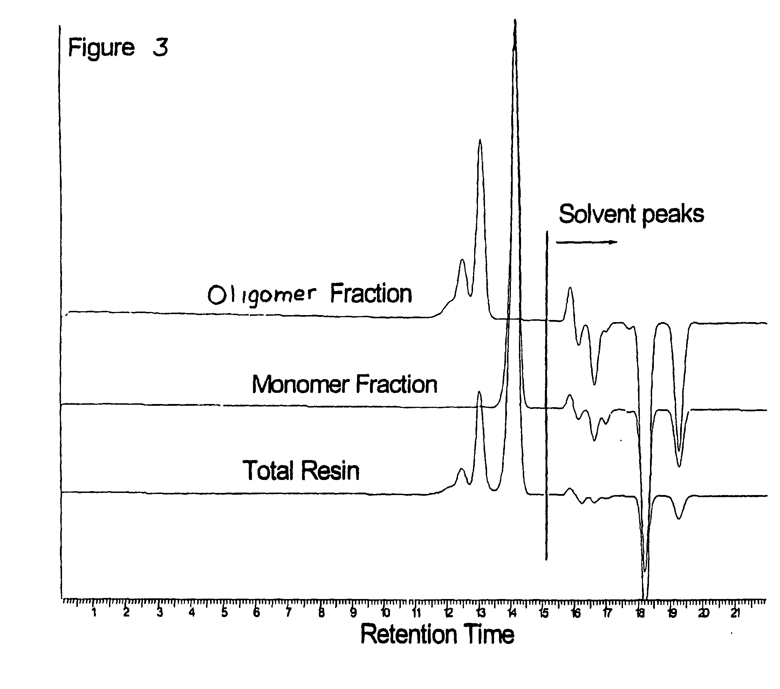

Figure 3 shows a series of three superimposed chromatograms of detected concentration of alkoxymethyl melamine component as a function of time (expressed in minutes). The oligomer components (the first two peaks) elute between about 12 and 13 minutes) and monomer component (the large peak) elutes between about 13 ½ and 14 ½ minutes). The lower chromatogram shows the separation of monomer, dimer and higher oligomers; the center chromatogram is a quality check of separated monomer; and the upper oligomer is a quality check of separated oligomer.

With reference to the lower chromatogram of Figure 3 a quantity of the dimer and greater oligomer fraction was collected beginning as the detector baseline began to rise above the noise level (baseline) and continued until approximately 15 seconds before the detection level reached the valley between the monomer and dimer peaks. A quantity of the monomer fraction was collected from between 15 seconds after the monomer peak began to rise above baseline until the detected monomer peak returned to baseline. Each of the samples was collected for a total of 10 runs of melamine solvent mixture through the SEC column.

The collected fractionated quantities of monomer and oligomer methyoxymethyl melamine were re-injected into a high pressure liquid chromatography (HPLC) system consisting of a Waters WISP 712 autosampler (75 µl injection), a Waters 590 programmable pump (1ml/min THF), a Waters 410 differential refractometer (sens = 64), using 2 Polymer Laboratories PL1gel, 50 Angstrom and 100 Angstrom, 5 micrometer columns. This was done to establish the purity of the monomer and oligomer samples. The quantities were considered acceptable when the area percent of the monomer peak was 99 wt. % or greater, assuming that the monomer and oligomer have a similar refractive index.

The methoxymethyl melamine material prepared as in Example 3 analyzed for an imino content of 0.13 wt. % and an oligomer content of 19 wt. %; after separation the monomer fraction analyzed for an imino content of 0.06 wt. % and the oligomer fraction analyzed for an imino content of 0.11 wt. %. The ratio of imino content of the monomer an oligomer fractions was about 0.5:1. The monomeric fraction was designated as substantially pure hexamethoxymethyl melamine (HMMM).

Cymel 300 (available from Cytec Inc) was analyzed as having an imino content of 0.45 wt. % and an oligomer content of 25 wt. %; after SEC fractionation, the monomer analyzed for an imino content of 0.25 wt. % and the oligomer analyzed for an imino content of 0.37 wt. %.

Example 7

This example serves to replicate aminoplast crosslinkers disclosed by Graziano et al in U.S. Patent 5,376,504. Cymel 300 was blended with HMMM as produced in Example 6 to provide an aminoplast resin containing 85 wt. % monomeric methoxymethyl melamine analyzed as having 0.31 wt. % imino and 15 wt. % oligomer.

### Uses of the melamine in low temperature cure coatings

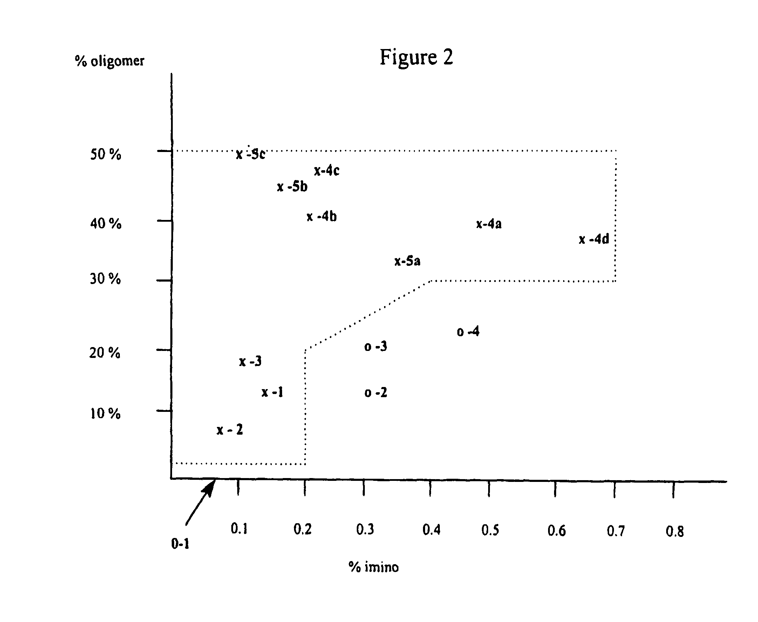

It has been surprisingly discovered that alkoxymethyl melamine according to this invention having low imino content and a wide range of oligomer content is useful for low temperature high quality crosslinked coatings. In the following Table 1 is a list of a variety of methoxymethyl and alkoxymethyl melamines used in the subsequent examples, where the designation "x-_" denotes a melamine according to this invention and "o-_" denotes a melamine of the prior art. See Figure 2 for a physical map of the melamines according to imino and oligomer content.

Table 1

| Figure 1 point | source | wt. % imino | wt. % oligomer |
|---|---|---|---|
| x-1 | Ex 1 | 0.13 | 15 |
| x-2 | Ex 2 | 0.08 | 7 |
| x-3 | Ex 3 | 0.11 | 19 |
| x-4a | Ex 4a | 0.47 | 40 |
| x-4b | Ex 4b | 0.21 | 42 |
| x-4c | Ex 4c | 0.23 | 47 |
| x-4d | Ex 4d | 0.66 | 40 |
| x-5a | Ex 5a | 0.36 | 36 |

Table 1   (continued)

| Figure 1 point | source | wt. % imino | wt. % oligomer |
|---|---|---|---|
| x-5b | Ex 5b | 0.18 | 45 |
| x-5c | Ex 5c | 0.11 | 50 |
| o-1 | Ex 6 | 0.06 | 0 |
| o-2 | Ex 7 | 0.31 | 15 |
| o-3 | CE-7103 (a) | 0.30 | 21 |
| o-4 | Cymel 300 (b) | 0.46 | 25 |

(a) CE-7103 is an alkoxymethyl melamine comprising mixed butyl and methyl ethers of methyl melamine; it is available from Solutia, Inc., St. Louis, Missouri as Resimene CE-7103.

(b) Cymel 300 is a methoxymethyl melamine which is available from Cytec Industries, Inc. of West Paterson, New Jersey.

Example 8

Coating Application with Methoxymethyl Melamine

This example serves to illustrate the use of a methoxymethyl melamine composition of this invention as crosslinker in low temperature curing coating formulations. A methoxymethyl melamine compound of this invention as prepared in Example 3 having 0.11 wt. %. imino content crosslinker and about 5.7 moles of methoxymethyl groups attached to pendant nitrogen atoms per mole of melamine and about 81 wt. %. monomeric alkoxymethyl melamine was mixed with acrylic polyol resin (available from Cook Chemicals & Composites, Kansas City, MO as Chempol-18-2500), 1,6-hexanediol, dimethyl succinate, propylene glycol methyl ether (PGME), methanol, acrylic polymer flow modifier (available from Solutia, Inc., St. Louis, MO as Modaflow "2100" and used as a 10 % solution in butyl acetate) and dodecylbenzene sulfonic acid ("DDBSA" used as a 20 % solution in methyl alcohol and isopropyl alcohol) in the proportions listed in Table 2.

Table 2

| Component | parts by weight |
|---|---|
| methoxymethyl melamine | 2.6 |
| acrylic polyol | 8.1 |
| hexanediol | 0.9 |
| dimethyl succinate | 0.12 |
| PGME | 0.7 |
| methanol | 0.5 |
| Modaflow 2100 | 0.34 |
| DDBSA | 0.21 |

The mixture was on coated onto an epoxy-coated aluminum panel to provide a dry film thickness of 0.025 mm (1 mil). The wet film was cured by 10 minute flash dry in air followed by curing for 30 minutes at 68 °C (155 °F) in a forced air oven.

A portion of the film was tested for solvent resistance by rubbing with methyl ethyl ketone (MEK) until the coating softens following the procedure of ASTM D-5402-93 "Standard Practice for Assessing the Solvent Resistance of Organic Coatings Using Solvent Rubs"; one MEK rub is a back and forth cycle. The oven-cured film failed at 66 MEK rubs.

A portion of the film was tested for hardness following the procedure of ASTM D 1474-98 "Standard Test Methods for Indentation Hardness of Organic Coatings" also known as "Knoop Hardness". The oven-cured film exhibited a Knoop Hardness of 6.4.

The film was allowed to age at ambient conditions for 72 hours and retested for solvent resistance and hardness. The aged film failed at 184 MEK rubs and exhibited a Knoop Hardness of 9.7.

Example 9

The procedure of Example 8 was repeated using the methoxymethyl melamine crosslinker as prepared in Example 1. The oven-cured film survived more than 200 MEK rubs and exhibited a Knoop Hardness of 7.9; a 72 hour-aged film survived more than 200 MEK rubs and exhibited a Knoop Hardness of 10.6.

Example 10

The procedure of Example 8 was essentially followed except the melamine compound was replaced with the methoxymethyl melamine prepared in Example 5a by supplemental methylation and having an imino content of about 0.36 wt. % and about 5.62 methoxymethyl groups attached to pendant nitrogen atoms per more of melamine. The oven-cured film failed at 122 MEK rubs and exhibited a Knoop Hardness of 6.0; the 72 hour aged film failed at 152 MEK rubs and exhibited a Knoop Hardness of 7.2.

Example 11

The procedure of Example 8 was essentially followed except the melamine compound was replaced with the methoxymethyl melamine prepared in Example 4a by supplemental methylation and having an imino content of about 0.47 wt. % and an oligomer content of about 40 wt. %. The oven-cured film failed at 37 MEK rubs and exhibited a Knoop Hardness of 1.2; the 72 hour aged film failed at 47 MEK rubs and exhibited a Knoop Hardness of 1.4.

Comparative Example 12

The procedure of Example 8 was essentially followed except the melamine compound was replaced with a commercially available methoxymethyl melamine (available from Cytec, Industries of West Paterson, New Jersey as Cymel 300) having an imino content of about 0.46 wt. %, and an oligomer content of 25 wt. %. The oven-cured film failed at 13 MEK rubs and exhibited a Knoop Hardness of <1; the 72 hour aged film failed at 18 MEK rubs and exhibited a Knoop Hardness of <1.

Example 13

Coating Application with Alkoxymethyl Melamine

This example serves to illustrate the use of an alkoxymethyl melamine composition of this invention as crosslinker in low temperature curing coating formulations. The procedure of Example 8 was essentially followed except that the melamine compound was replaced with 2.84 parts of an alkoxymethyl melamine compound of this invention prepared according to Example 2. The oven-cured film failed at 200 MEK rubs and exhibited a Knoop Hardness of 6.83; a 72 hour-aged film survived over 200 MEK rubs and exhibited a Knoop Hardness of 10.6.

Comparative Example 14

The process of Example 9 was repeated to produce a coating cured at 68 °C using a commercial alkoxymethyl melamine, designated CE-7103 having an imino content of 0.3 wt. % and an oligomer content of 21 wt. %. The oven cured film failed at 9 MEK rubs and exhibited a Knoop Hardness of <1; a 72 hour-aged film failed at 53 MEK rubs and exhibited a Knoop Hardness of <1.

Comparative Example 15

The procedure of Example 8 was essentially followed except the melamine compound was replaced with a commercially available alkoxymethyl melamine (obtained from Solutia, Inc. as Resimene RF-4518) having an imino content of about 0.28 wt. %and a total of about 5.0 alkoxymethyl groups attached to pendant nitrogen atoms per mole of melamine. The oven-cured film failed at 23 MEK rubs and exhibited a Knoop Hardness of <1; the 72 hour aged film failed at 31 MEK rubs and exhibited a Knoop Hardness of <1. The results of the coating applications are summarized in Table 3 which shows the advantage of alkoxymethyl (including methoxymethyl) melamine compounds having a balance of low imino and high alkylation in providing crosslinker for low temperature curing coating formulations.

Table 3

| Example | imino content | oligomer content | solvent resistance* | hardness* |
|---|---|---|---|---|
| 8 | 0.11 wt. % | 19 wt. % | 66/184 MEK rubs | 6.4/9.7 Knoop |
| 9 | 0.13 | 15 | 200/200+ | 7.9/10.6 |
| 10 | 0.36 | 36 | 122/152 | 6.0/7.2 |

* MEK rubs and Knoop Hardness reported for "oven-cured"/"72 hour -aged" films

Table 3   (continued)

| Example | imino content | oligomer content | solvent resistance* | hardness* |
|---|---|---|---|---|
| 11 | 0.47 | 40 | 37/47 | 1.2/1.47 |
| comp 12 | 0.46 | 25 | 13/18 | <1/<1 |
| 13 | 0.08 | 7 | 200/200+ | 6.8/9.7 |
| comp 14 | 0.3 | 21 | 9/53 | <1/<1 |
| comp 15 | 0.28 | | 23/31 | <1/<1 |

* MEK rubs and Knoop Hardness reported for "oven-cured"/"72 hour -aged" films

Example 16

This examples serves to illustrate the advantages of high oligomeric, low imino content methoxymethyl melamine compositions of this invention as crosslinker in low temperature curing coating formulations. A variety of crosslinker materials according to this invention and the prior art were used to prepare films of crosslinked coating composition following the procedure of Example 8 except the oven cure temperature was at 71, 77 and 82 °C. The 72 hour Knoop Hardness is reported in Table 4.

Table 4

| Source | imino | oligomer | Knoop Hardness | | |
|---|---|---|---|---|---|
| | | | 71 | 77 | 82 |
| Ex 5b | 0.18 wt. %. | 45 wt. %. | 13.8 | 15.4 | 16.3 |
| Ex 5c | 0.11 | 50 | 5.25 | 9.83 | 13.9 |
| Ex 6 | 0.06 | 0 | 16.5 | 16.5 | 18.5 |
| Ex 3 | 0.11 | 19 | 15.6 | 18.1 | 19.0 |
| Ex 1 | 0.13 | 15 | 14.2 | 16.9 | 17.1 |

Example 17

This example serves to illustrate the advantages of other high oligomeric, low imino content methoxymethyl melamine compositions of this invention as crosslinker in low temperature curing coating formulations. A variety of crosslinker materials according to this invention and the prior art were used to prepare films of crosslinked coating composition following the procedure of Example 8 except the oven cure temperature was at 71, 77 and 82 °C. The 72 hour Knoop Hardness is reported in Table 5.

Table 5

| Source | imino | oligomer | Knoop Hardness | | |
|---|---|---|---|---|---|
| | | | 71 | 77 | 82 |
| Ex 4a | 0.46 wt. %. | 40 wt. %. | 1.0 | 2.4 | 6.7 |
| Ex 4b | 0.21 | 42 | 2.8 | 7.0 | 12.6 |
| Ex 4c | 0.23 | 47 | 2.0 | 4.9 | 10.4 |
| --- | ~1.0 | --- | --- | --- | 0.6 |
| --- | 0.46 | 25 | 0.6 | 1.3 | 1.8 |

**Claims**

1.   A crosslinker composition consisting essentially of

(a) 50 to 95 weight percent monomeric $C_1$ to $C_8$ alkoxymethyl melamine derivatives containing not more than 0.20 wt. % of imino (>N-H) groups; and
(b) 5 to 50 weight percent oligomeric $C_1$ to $C_8$ alkoxymethyl melamine derivatives, wherein

(i) when said composition comprises from 5 to 20 wt. % oligomer, said composition has an imino content of less than 0.20 wt. %;

(ii) when said composition comprises from 20 to 30 wt. % oligomer, said composition has an imino content, I, defined by the algorithm, $I \leq 0.02X - 0.2$, where X is the weight percent oligomer in the composition and I is expressed in weight percent imino;

(iii) when said composition comprises from 30 to 50 wt. % oligomer, said composition has an imino content of less than 0.7 wt. %.

2. The composition according to claim 1 which is liquid at 20 °C.

3. The composition according to claim 1 wherein said composition has an imino content of less than 0.6 wt. %.

4. The composition according to claim 1 wherein said composition has an imino content of less than 0.5 wt. %.

5. The composition according to claim 1 wherein said composition has an imino content of less than 0.4 wt. %.

6. The composition according to claim 1 wherein said composition has an imino content of less than 0.3 wt. %.

7. The composition according to claim 1 wherein said composition has an imino content of less than 0.2 wt. %.

8. The composition according to claim 1 wherein said when said alkoxymethyl melamine derivatives are methoxymethyl melamine derivatives.

9. The composition according to claim 8 which is liquid at 20 °C.

10. The composition according to claim 8 wherein said composition has an imino content of less than 0.6 wt. %.

11. The composition according to claim 8 wherein said composition has an imino content of less than 0.5 wt. %.

12. The composition according to claim 8 wherein said composition has an imino content of less than 0.4 wt. %.

13. The composition according to claim 8 wherein said composition has an imino content of less than 0.3 wt. %.

14. The composition according to claim 8 wherein said composition has an imino content of less than 0.2 wt. %.

15. The composition according to claim 1 wherein for each mole of melamine in the melamine derivatives in said composition there is at least 5.6 moles of alkoxymethyl groups attached to pendant nitrogen atoms of said melamine, where the alkoxymethyl groups are mixtures of methyoxymethyl and minor amounts higher alkoxymethyl groups; where the amount of higher alkoxymethyl groups present does not inhibit curing of a standard coating at 66 °C to a hardness which survives at least 30 MEK rubs.

16. A crosslinker composition comprising monomeric and oligomeric alkoxymethylated melamine, wherein monomeric alkoxymethylated melamine molecules have 6 moles of substituent groups attached to pendant nitrogen atoms per mole of monomeric melamine, wherein said substituent groups are selected from the group consisting of imino [>N-H], methylol [>N-CH$_2$OH], alkoxymethyl [>N-CH$_2$OR] and acetal [>N-CH$_2$OCH$_2$OR]; and wherein difunctional bridging groups between melamine units in oligomeric alkoxymethylated melamine are selected from the group consisting of methylene groups [>N-CH$_2$-N<] and methylene ether [>N-CH$_2$OCH$_2$-N<] groups; wherein:

(a) monomeric alkoxymethylated melamine units comprise at least 50 and up to 95 percent by weight of the monomeric and oligomeric alkoxymethylated melamine units in the composition as determined by size exclusion chromatography,

(b) alkoxymethyl groups comprise at least 5.0 moles of substituent groups attached to pendant nitrogen atoms per mole of monomeric melamine,

(c) said alkoxymethyl groups on each melamine unit are methoxymethyl or mixtures of methyoxymethyl and higher alkoxymethyl groups;

(d) when said composition comprises from 5 to 20 wt. % oligomer, said composition has an imino content of less than 0.20 wt. %;

(e) when said composition comprises from 20 to 30 wt. % oligomer, said composition has an imino content, I, defined by the algorithm, $I \leq 0.02X - 0.2$, where X is the weight percent oligomer in the composition and I is expressed in weight percent imino;
(f) when said composition comprises from 30 to 50 wt. % oligomer, said composition has an imino content of less than 0.7 wt. %.

**Patentansprüche**

1. Vernetzer-Zusammensetzung bestehend im wesentlichen aus Massenanteilen, bezogen auf die Masse der Zusammensetzung, von

   (a) 50 % bis 95 % von monomeren $C_1$- bis $C_8$- Alkoxymethylmelamin-Derivaten enthaltend einen Massenanteil von nicht mehr als 0,20 % an Iminogruppen der Formel > N-H, und

   (b) 5 % bis 50 % von oligomeren $C_1$- bis $C_8$- Alkoxymethylmelamin-Derivaten,

   wobei

   (i) die besagte Zusammensetzung einen Massenanteil an Iminogruppen von weniger als 0,20 % aufweist, wenn die Zusammensetzung einen Massenanteil von 5 % bis 20 % an Oligomeren enthält,

   (ii) die besagte Zusammensetzung einen Massenanteil I, gemessen in %, an Iminogruppen aufweist, der durch die Formel $I \leq 0,02 \times X - 0,2$ definiert ist, wobei X der Massenanteil an Oligomeren in der Zusammensetzung ist, gemessen in %, falls die Zusammensetzung einen Massenanteil X von 20 % bis 30 % an Oligomeren enthält,

   (iii) die besagte Zusammensetzung einen Massenanteil an Iminogruppen von weniger als 0,7 % aufweist, wenn die Zusammensetzung einen Massenanteil von 30 % bis 50 % an Oligomeren enthält.

2. Zusammensetzung gemäß Anspruch 1, die bei 20 °C flüssig ist.

3. Zusammensetzung gemäß Anspruch 1, wobei die besagte Zusammensetzung einen Massenanteil an Iminogruppen von weniger als 0,6 % aufweist.

4. Zusammensetzung gemäß Anspruch 1, wobei die besagte Zusammensetzung einen Massenanteil an Iminogruppen von weniger als 0,5 % aufweist.

5. Zusammensetzung gemäß Anspruch 1, wobei die besagte Zusammensetzung einen Massenanteil an Iminogruppen von weniger als 0,4 % aufweist.

6. Zusammensetzung gemäß Anspruch 1, wobei die besagte Zusammensetzung einen Massenanteil an Iminogruppen von weniger als 0,3 % aufweist.

7. Zusammensetzung gemäß Anspruch 1, wobei die besagte Zusammensetzung einen Massenanteil an Iminogruppen von weniger als 0,2 % aufweist.

8. Zusammensetzung gemäß Anspruch 1, wobei die besagten Alkoxymethylmelamin-Derivate Methoxymethylmelamin-Derivate sind.

9. Zusammensetzung gemäß Anspruch 8, die bei 20 °C flüssig ist.

10. Zusammensetzung gemäß Anspruch 8, wobei die besagte Zusammensetzung einen Massenanteil an Iminogruppen von weniger als 0,6 % aufweist.

11. Zusammensetzung gemäß Anspruch 8, wobei die besagte Zusammensetzung einen Massenanteil an Iminogruppen von weniger als 0,5 % aufweist.

12. Zusammensetzung gemäß Anspruch 8, wobei die besagte Zusammensetzung einen Massenanteil an Iminogruppen von weniger als 0,4 % aufweist.

**13.** Zusammensetzung gemäß Anspruch 8, wobei die besagte Zusammensetzung einen Massenanteil an Iminogruppen von weniger als 0,3 % aufweist.

**14.** Zusammensetzung gemäß Anspruch 8, wobei die besagte Zusammensetzung einen Massenanteil an Iminogruppen von weniger als 0,2 % aufweist.

**15.** Zusammensetzung gemäß Anspruch 1, bei der für jeweils 1 mol Melamin in den Melamin-Derivaten mindestens 5,6 mol an Alkoxymethylgruppen mit den nicht ringständigen Stickstoffatomen des besagten Melamins verbunden sind, wobei die Alkoxymethylgruppen Mischungen von Methoxymethylgruppen und geringen Mengen von höheren Alkoxymethylgruppen sind, und wobei die Menge an höheren Alkoxymethylgruppen nicht hinderlich ist für die Vernetzung einer Standardbeschichtung bei 66 °C zu einer solchen Härte, die mindestens 30 Wischungen mit Methyläthylketon übersteht.

**16.** Vernetzer-Zusammensetzung enthaltend monomere und oligomere alkoxymethylierte Melamin-Derivate; wobei monomere alkoxymethylierte Melamin-Moleküle 6 mol an Substituentengruppen je 1 mol des monomeren Melamins aufweisen, die mit den nicht ringständigen Stickstoffatomen verbunden sind, die besagten Substituentengruppen ausgewählt sind aus der Gruppe der Iminogruppen > N-H, der Methylolgruppen > N-CH$_2$-OH, der Alkoxymethylgruppen >N-CH$_2$-OR, und der Acetalgruppen >N-CH$_2$O-CH$_2$-OR; und wobei difunktionelle Brückengruppen zwischen Melamin-Einheiten in oligomeren alkoxymethylierten Melaminen ausgewählt sind aus der Gruppe der Methylengruppen > N-CH$_2$-N < und der Methylenäthergruppen > N-CH$_2$-O-CH$_2$-N < ; wobei

(a) monomere alkoxymethylierte Melamin-Einheiten einen Massenanteil von mindestens 50 % und bis zu 90 % von der Masse der monomeren und oligomeren alkoxymethylierten Melaminen in der Zusammensetzung bilden, bestimmt durch Ausschlußchromatographie,

(b) Alkoxymethylgruppen mindestens 5,0 mol, bezogen auf 1 mol des monomeren Melamins, derjenigen Substituentengruppen sind, die mit den nicht ringständigen Stickstoffatomen verbunden sind,

(c) die besagten Alkoxymethylgruppen an jeder Melamin-Einheit Methoxymethylgruppen oder Mischungen von Methoxymethylgruppen und höheren Alkoxymethylgruppen sind,

(d) die besagte Zusammensetzung einen Massenanteil an Iminogruppen von weniger als 0,20 % aufweist, falls die Zusammensetzung einen Massenanteil von 5 % bis 20 % an Oligomeren enthält,

(e) die besagte Zusammensetzung einen Massenanteil I, gemessen in %, an Iminogruppen aufweist, der durch die Formel I ≤ 0,02 x X - 0,2 definiert ist, wobei X der Massenanteil an Oligomeren in der Zusammensetzung ist, gemessen in %, falls die Zusammensetzung einen Massenanteil X von 20 % bis 30 % an Oligomeren enthält,

(f) die besagte Zusammensetzung einen Massenanteil an Iminogruppen von weniger als 0,7 % aufweist, wenn die Zusammensetzung einen Massenanteil von 30 % bis 50 % an Oligomeren enthält.

**Revendications**

**1.** Composition réticulante comprenant essentiellement des fractions massiques de

(a) 50 % à 95 % de dérivés monomériques de C$_1$- à C$_8$ alkoxyméthyle mélamine comprenant une fraction massique n'excédant pas 0,20 % de groupes imino, >N-H, et

(b) 5 % à 50 % de dérivés oligomériques de C$_1$- à C$_8$- alkoxyméthyle mélamine,

**caractérisée en ce que**

(i) la fraction massique de groupes imino dans cette composition est moins de 0,20 % si cette composition contient une fraction massique entre 5 % et 20 % d'oligomères,

(ii) la fraction massique I, en %, de groupes imino est définie par la formule I ≤ 0,02 x X - 0,2, X désignant la fraction massique en %, d'oligomères présent dans la composition, si cette composition contient une fraction massique entre 20 % et 30 % d'oligomères,

(iii) la fraction massique de groupes imino dans cette composition est moins de 0,70 % si cette composition contient une fraction massique entre 30 % et 50 % d'oligomères.

2.  Composition selon la revendication 1, **caractérisée en ce qu'**elle est liquide à 20°C.

3.  Composition selon la revendication 1, **caractérisée en ce que** la fraction massique de groupes imino dans cette composition est moins de 0,6 %.

4.  Composition selon la revendication 1, **caractérisée en ce que** la fraction massique de groupes imino dans cette composition est moins de 0,5 %.

5.  Composition selon la revendication 1, **caractérisée en ce que** la fraction massique de groupes imino dans cette composition est moins de 0,4 %.

6.  Composition selon la revendication 1, **caractérisée en ce que** la fraction massique de groupes imino dans cette composition est moins de 0,3 %.

7.  Composition selon la revendication 1, **caractérisée en ce que** la fraction massique de groupes imino dans cette composition est moins de 0,2 %.

8.  Composition selon la revendication 1, **caractérisée en ce que** lesdits dérivés alkoxyméthyle de mélamine sont des dérivés méthoxyméthyle de mélamine.

9.  Composition selon la revendication 8, **caractérisée en ce que** elle est liquide à 20 °C.

10. Composition selon la revendication 8, **caractérisée en ce que** la fraction massique de groupes imino dans cette composition est moins de 0,6 %.

11. Composition selon la revendication 8, **caractérisée en ce que** la fraction massique de groupes imino dans cette composition est moins de 0,5 %.

12. Composition selon la revendication 8, **caractérisée en ce que** la fraction massique de groupes imino dans cette composition est moins de 0,4 %.

13. Composition selon la revendication 8, **caractérisée en ce que** la fraction massique de groupes imino dans cette composition est moins de 0,3 %.

14. Composition selon la revendication 8, **caractérisée en ce que** la fraction massique de groupes imino dans cette composition est moins de 0,2 %.

15. Composition selon la revendication 1, **caractérisée en ce que** pour chaque 1 mol de mélamine dans les dérivés de mélamine dans ladite composition, il y a au moins 5,6 mol de groupes alkoxyméthyle attachés aux atomes nitrogène qui ne font pas parti du cycle triazinique de cette mélamine, et que les groupes alkoxyméthyle sont des mélanges de groupes méthoxyméthyle et des quantités mineures de groupes alkoxyméthyle avec plusieurs atomes de carbone dans les radicaux alkyles, ce taux des derniers groupes ne pas empêchant la réticulation d'un revêtement standard à 66 °C à un degré de cuisson qui survit au moins 30 rubs dans l'essai MEK.

16. Composition réticulante comprenant de mélamines alkoxyméthylées monomériques et oligomériques, **caractérisée en ce que** les molécules de mélamine alkoxyméthylée monomériques ont 6 mol de groupes substituants attachés aux atomes nitrogènes qui ne font pas parti du cycle triazinique, ces groupes étant choisis parmi les groupes imino > N-H, méthylole > N-CH$_2$-OH, alkoxyméthyle > N-CH$_2$-OR, et acétale > N-CH$_2$-O-CH$_2$-OR; et **en ce que** les groupes difonctionnels reliant les unités de mélamine dans les mélamines alkoxyméthylées oligomériques sont choisis parmi les groupes méthylène >N-CH$_2$N< et éther méthylénique > N-CH$_2$-O-CH$_2$-N <, et **en ce que**

(a) les unités de mélamine alkoxyméthylée monomériques présentent une fraction massique d'au moins 50 %, et jusqu'à 95 % dans le total des unités de mélamine alkoxyméthylée monomériques et oligomériques, déterminé par chromatographie sur gels,

(b) des groupes alkoxyméthyle présentent au moins 5,0 mol sur 1 mol de mélamine, des groupes substituant attachés aux atomes nitrogène qui ne font pas parti du cycle triazinique,

(c) lesdits groupes alkoxyméthyle de chaque unité de mélamine sont des groupes méthoxyméthyle ou des mélanges des groupes méthoxyméthyle avec des groupes alkoxyméthyle avec plusieurs atomes de carbone dans les radicaux alkyles,

(d) la fraction massique de groupes imino dans cette composition est moins de 0,20 % si cette composition contient une fraction massique entre 5 % et 20 % d'oligomères,

(e) la fraction massique I, en %, de groupes imino est définie par la formule $I \leq 0,02 \times X - 0,2$, X désignant la fraction massique en %, d'oligomères présent dans la composition, si cette composition contient une fraction massique entre 20 % et 30 % d'oligomères,

(f) la fraction massique de groupes imino dans cette composition est moins de 0,70 % si cette composition contient une fraction massique entre 30 % et 50 % d'oligomères.

FIGURE 1

Figure 2

EP 1 268 592 B1

Figure 3

Oligomer Fraction

Monomer Fraction

Total Resin

Solvent peaks

Retention Time

EP 1 268 592 B1